# EUROPÄISCHE PATENTSCHRIFT

(11) **EP 0 281 791 B1**
(45) Veröffentlichungstag und Bekanntmachung des Hinweises auf die Patenterteilung: **03.02.1993**
(21) Anmeldenummer: 88102144.8
(22) Anmeldetag: 13.02.1988
(51) Int. Cl.: C12Q 1/56

(54) **Verfahren zur Bestimmung von Protein C-Inhibitor**
Method for determining a protein C inhibitor
Procédé pour la détermination d'inhibiteur de protéine C

(30) Priorität: 23.02.1987 DE 3705744
(43) Veröffentlichungstag der Anmeldung: 14.09.1988
(73) Patentinhaber: BEHRINGWERKE Aktiengesellschaft, 35001 Marburg (DE)
(72) Erfinder: Stief, Thomas, Dr., D-3550 Marburg (DE); Heimburger, Norbert, Prof. Dr., D-3550 Marburg (DE); Radtke, Klaus-Peter, Dr., D-3550 Marburg (DE)

(56) Entgegenhaltungen:
- EP-A- 0 189 910
- EP-A- 0 198 322
- WO-A-86/00413
- BIOLOGICAL ABSTRACTS/RRM, Band 34, 1987; M.J.HEEB et al., Nr. 10355*
- CLINICAL CHEMISTRY, Band 32, 1986; J.CHMIELEWSKA et al., Seiten 482-485*

## Beschreibung

Die Erfindung betrifft ein Verfahren zur Bestimmung der Protein C-Inhibitor-Aktivität in einer Flüssigkeit ("funktionelle" Bestimmung des Protein C-Inhibitors), worin der Protein C-Inhibitor auf eine Plasminogen-Aktivator-Menge bekannter Aktivität einwirkt und einen Teil dieser Aktivität hemmt und aus der Restaktivität des Plasminogen-Aktivators die Protein C-Inhibitor-Aktivität durch Zugabe eines chromogenen Substrates bestimmt wird, indem die durch das Auftreten des Chromophors entstehende Änderung der Extinktion mit einer spektrophotometrischen Methode gemessen wird.

Protein C-Inhibitor ist ein noch nicht sehr lange bekannter Inhibitor für aktiviertes Protein C. Aktiviertes Protein C, eine Vitamin K-abhängige Serinprotease entsteht, wenn Protein C im Verlauf der Gerinnungsaktivierung durch den Komplex Thrombin-Thrombomodulin aktiviert wird. Protein C ist das zentrale Protein in einer der wichtigsten Regulationsmechanismen der Haemostase. Aktiviertes Protein C verringert auf der einen Seite die Bildungsrate von Thrombin, indem es die Gerinnungsfaktoren F Va und F VIIIa inaktiviert. Auf der anderen Seite stimuliert es die Fibrinolyse. Sowohl die antikoagulatorische als auch die profibrinolytische Funktion von Protein C werden durch den Protein C-Inhibitor kontrolliert, der damit eine wichtige Funktion bei der Kontrolle der Antikoagulation und Fibrinolyse hat. Aus diesem Grund ist eine funktionelle Bestimmung von Protein C-Inhibitor von großer Bedeutung.

Funktionelle Bestimmungen von Protein C-Inhibitor nach dem Stand der Technik benutzen ein Verfahren, bei dem die inhibitorische Wirkung von Protein C-Inhibitor auf aktitiviertes Protein C ausgenutzt wird. (J. Biol. Chem. 258 (1) 163-168, 1983). Chmielewska, J. und Wiman, B. (1986), Clin. Chem. 32, 482-485, beschreiben ein Verfahren, in dem eine Körperflüssigkeit mit einer definierten Menge eines Plasminogenaktivators inkubiert und danach die Restaktivität des Plasminogenaktivators bestimmt wird.

Es wurde nun überraschenderweise gefunden, daß Protein C-Inhibitor nicht nur aktiviertes Protein C hemmt, sondern auch die Plasminogen-Aktivatoren Urokinase und Gewebsplasminogen-Aktivator. Die Inhibition der Plasminogenaktivatoren wird in Gegenwart von Heparin oder einem anderen polysulfatierten Polysaccharid beschleunigt.

Es wurde weiterhin überrrascenderweise gefunden, daß die Plasminogen-Aktivatoren Urokinase und Gewebsplasminogen-Aktivator, vorzugsweise Urokinase, anstelle von aktiviertem Protein C für die diagnostische Bestimmung von Protein C-Inhibitor angewendet werden können.

Weiter wurde gefunden, daß bei Anwendung des erfindungsgemäßen Verfahrens die Protein C-Inhibitor-Bestimmung in Plasma durchgeführt werden kann, wenn die, ebenfalls durch Heparin oder ein anderes polysulfatiertes Polysaccharid katalysierte, Hemmung von Urokinase durch Antithrombin III ausgeschaltet wird. Überraschenderweise wurde gefunden, daß dies erreicht werden kann, wenn dem Reaktionsansatz Fibrinogen zugesetzt wird, man Heparin durch Dextransulfat oder Pentosansulfat ersetzt und die Inkubation des Plasminogen-Aktivators bei 5-40°C, vorzugsweise 10-30°C, besonders bevorzugt 23°C durchgeführt wird. Unter diesen Reaktionsbedingungen verläuft die Hemmung von Urokinase durch den Protein C-Inhibitor mit der gleichen Geschwindigkeit wie bei der Verwendung von Heparin als Kofaktor.

Gegenstand der Erfindung ist daher ein Verfahren zur funktionellen Bestimmung von Protein C-Inhibitor in einer Korperflüssigkeit nach einem der Ansprüche 1-7.

Die Aktivität des Plasminogenaktivators kann mittels eines für diesen Plasminogenaktivator spezifischen chromogenen Substrates bestimmt werden. Die Veränderung der Extinktion, hervorgerufen durch die Abspaltung des Chromophors wird mit einer spektrophotometrischen Methode gemessen. Sie ist der Konzentration des Plasminogenaktivators proportional.

Vergleiche ergaben eine gute Übereinstimmung zwischen der Protein C-Inhibitor-Bestimmung gemäß der Erfindung und einer Protein C-Inhibitor-Bestimmung nach dem Stand der Technik. Letztere wurde in Gegenwart von aktiviertem Protein C durchgeführt, aus dessen Aktivität die Protein C-Inhibitor-Konzentration ermittelt wurde.

Gegenstand der Erfindung ist auch ein Mittel nach Anspruch 8 zur Verwendung in dem erfindungsgemäßen Verfahren.

Zur Herstellung eines zur Verwendung in dem erfindungsgemäßen Verfahren geeigneten Mittels, das Plasminogenaktivator enthält, werden beispielsweise humane Urokinase in einer Konzentration von 0,1-5000, vorzugsweise 10-1000 IE/ml oder Gewebsplasminogen-Aktivator in einer Konzentration von 0,001-500, vorzugsweise 0,1-10 µg/ml in einer Pufferlösung, vorzugsweise 25-250 mmol/l Tris-Pufferlösung, besonders 50 mmol/l Tris, pH 6 - 9, vorzugsweise pH 8, gelöst.

Dextransulfat ist in einer Konzentration von 10⁻⁷-10⁻¹ g/l, vorzugsweise 1,5 x 10⁻³g/l und Fibrinogen in einer Konzentration von 5 x 10⁻⁵- 5 mg/ml, vorzugsweise 0,25 mg/ml in einem solchen Mittel enthalten.

Weiterhin enthält ein solches Mittel zweckmäßigerweise 50 - 250 mmol/l, vorzugsweise 80 - 120 mmol/l NaCl, 2 - 20 g/l, vorzugsweise 10 g/l einer abgebauten und chemisch vernetzten Gelatine siehe DE-A-1118792, sowie 0,5 - 5 g/l, vorzugsweise 1 g/l eines nichtionischen Detergenzes.

Gegebenenfalls kann das Mittel durch Zusätze stabilisiert und lyophilisiert werden.

Folgendes Beispiel erläutert die Erfindung:

### Beipiel

### a) Herstellung eines für das erfindungsgemäße Verfahren geeigneten Mittels

Humane Urokinase (Actosolv^{(R)}, Behringwerke AG, Marburg) wurde in 50 mmol/l Tris-Pufferlösung, pH 8 gelöst. Die Endkonzentration an Urokinase betrug 200 IE/ml.

Es wurden 1,5 x 10⁻³ g/l Dextransulfat (MW ca. 8000, Sulfatierungsgrad ca. 20 %) 0,25 mg/ml Fibrinogen, 100 mmol/l NaCl, 10 g/l Polygelin (Behringwerke AG, Marburg) und 1 g/l ^{R}Triton X 100 zugegeben.

Die Urokinase kann durch 1 µg/ml Gewebsplasminogen-Aktivator ersetzt werden (230 IE/ml).

### b) Nachweis von Protein C-Inhibitor in Pufferlösungen

100 µl des unter a) beschriebenen Mittels wurden mit 50 µl Plasma 2 Minuten lang bei Raumtemperatur inkubiert. Nach Zugabe von 500 µl Substratlösung (S2444 ^{(R)}Kabi Vitrum, 0,3 mmol/l in 50 mmol/l Trispuffer) folgte eine weitere Inkubationszeit von 30 Minuten bei 37° C. Durch Zugabe von 100 µl 8,5 mol/l Essigsäure wurde die Substratumsetzung gestoppt und die Extinktion der Probe in einem Photometer (405 nm) bestimmt. Die Auswertung der Meßergebnisse erfolgte über eine Bezugskurve, die durch Zugabe von Urokinase zu einem Protein C-Inhibitor-Mangelpuffer erhalten wurde. Die gemessene Urokinase-Konzentration verhielt sich dann zur Protein C-Inhibitor-Konzentration umgekehrt proportional. Das Volumen des Urokinase-Reagenzes kann zwischen 50 - 200 µl, vorzugsweise 100 µl, betragen. Die Inkubationszeit des Reagenzes mit der Probe kann zwischen 2 und 20 Minuten variiert werden.

### c) Nachweis von Protein C-Inhibitor im Plasma über chromogenes Urokinasesubstrat oder Plasminogen und chromogenes Plasminsubstrat

100 µl des unter a) beschriebenen Mittels wurden mit 50 µl Plasma 2 Minuten lang bei Raumtemperatur (23°C) inkubiert.

Nach Zugabe von 100 µl 0,5 M Natriumacetatpuffer, pH 4,0, wurde 20 Minuten bei 37°C inkubiert. Neutralisiert wurde der Testansatz daraufhin durch Zugabe von 500 µl Substratlösung (S 2444, ^{(R)}Kabi Vitrum, 0,3 mmol/l in 250 mmol/l Trispuffer, pH 9,6). Nach einer weiteren Inkubationszeit von 30 Minuten bei 37°C wurde die Substratumsetzung durch Zusatz von 100 µl 8,5 mol/l Essigsäure gestoppt und die Extinktion der Probe in einem Photometer (405 nm) bestimmt.

In einer Variation dieses Beispiels wurde das chromogene Substrat S 2444 durch Plasminogen ( 2 CTA/ml) und das chromogene Plasminsubstrat BCP 500 Behringwerke AG, Marburg (0,3 mmol/l) in Trispuffer (250 mmol/l, pH 9,6) ersetzt und die Endinkubationszeit auf 1 Minute verkürzt.

Die Auswertung der Meßergebnisse erfolgt wie im Beispiel b) angegeben. Anstelle der Protein C-Inhibitor-Mangelpufferlösung wird Protein C-Inhibitor-Mangelplasma verwendet.

Dieses Plasma wurde über eine Anti-Protein C-Inhibitor-Sepharose immunadsorptiv hergestellt.

## Patentansprüche (Patentansprüche für folgende(n) Vertragsstaat(en): AT, BE, CH, DE, FR, GB, IT, LI, LU, NL, SE)

1. Verfahren zur funktionellen Bestimmung von Protein C-Inhibitor in einer Probe einer Körperflüssigkeit, dadurch gekennzeichnet, daß diese Probe mit einer definierten Menge eines Plasminogenaktivators vorinkubiert wird und dann die Restaktivität des Plasminogenaktivators bestimmt wird, wobei die Vorinkubation in Gegenwart von Dextransulfat und Fibrinogen erfolgt.

2. Verfahren nach Anspruch 1, dadurch gekennzeichnet, daß als Plasminogenaktivator Urokinase verwendet wird.

3. Verfahren nach Anspruch 1, dadurch gekennzeichnet, daß Gewebsplasminogenaktivator verwendet wird.

4. Verfahren nach Anspruch 1, dadurch gekennzeichnet, daß das Dextransulfat in einer Konzentration von 10⁻⁸ bis 1 mg/ml im Testansatz ist.

5. Verfahren nach Anspruch 1, dadurch gekennzeichnet, daß die Restaktivität des Plasminogenaktivators mittels eines für diesen Plasminogenaktivator spezifischen chromogenen Substrates bestimmt wird.

6. Verfahren nach Anspruch 1, dadurch gekennzeichnet, daß die Restaktivität des Plasminogenaktivators mittels Plasminogen und eines chromogenen Plasminsubstrats bestimmt wird.

7. Verfahren nach Anspruch 1, dadurch gekennzeichnet, daß die Bestimmung bei 20-25 °C durchgeführt wird.

8. Mittel enthaltend 0,1-5000 IE/ml Urokinase, 10⁻⁷-10⁻¹ g/l Dextransulfat, 5 x 10⁻⁵ bis 5 mg/ml Fibrinogen, 2-20 g/l einer abgebauten und chemisch vernetzten Gelatine und 0,5-5 g/l einer nichtionischen Detergenz in einer Pufferlösung pH 6-9.

## Patentansprüche (Patentansprüche für folgende(n) Vertragsstaat(en): ES)

1. Verfahren zur funktionellen Bestimmung von Protein C-Inhibitor in einer Probe einer Körperflüssigkeit, dadurch gekennzeichnet, daß diese Probe mit einer definierten Menge eines Plasminogenaktivators vorinkubiert wird und dann die Restaktivität des Plasminogenaktivators bestimmt wird, wobei die Vorinkubation in Gegenwart von Dextransulfat und Fibrinogen erfolgt.

2. Verfahren nach Anspruch 1, dadurch gekennzeichnet, daß als Plasminogenaktivator Urokinase verwendet wird.

3. Verfahren nach Anspruch 1, dadurch gekennzeichnet, daß Gewebsplasminogenaktivator verwendet wird.

4. Verfahren nach Anspruch 1, dadurch gekennzeichnet, daß das Dextransulfat in einer Konzentration von 10⁻⁸ bis 1 mg/ml im Testansatz ist.

5. Verfahren nach Anspruch 1, dadurch gekennzeichnet, daß die Restaktivität des Plasminogenaktivators mittels eines für diesen Plasminogenaktivator spezifischen chromogenen Substrates bestimmt wird.

6. Verfahren nach Anspruch 1, dadurch gekennzeichnet, daß die Restaktivität des Plasminogenaktivators mittels Plasminogen und eines chromogenen Plasminsubstrats bestimmt wird.

7. Verfahren nach Anspruch 1, dadurch gekennzeichnet, daß die Bestimmung bei 20-25 °C durchgeführt wird.

## Claims (Claims for the following Contracting State(s): AT, BE, CH, DE, FR, GB, IT, LI, LU, NL, SE)

1. A method for the functional determination of protein C inhibitor in a body fluid sample, which comprises prior incubation of this sample with a defined amount of a plasminogen activator, and then determination of the residual activity of the plasminogen activator, the prior incubation being carried out in the presence of dextran sulfate and fibrinogen.

2. The method as claimed in claim 1, wherein urokinase is used as plasminogen activator.

3. The method as claimed in claim 1, wherein tissue plasminogen activator is used.

4. The method as claimed in claim 1, wherein the dextran sulfate is in a concentration of 10⁻⁸ to 1 mg/ml in the test batch.

5. The method as claimed in claim 1, wherein the residual activity of the plasminogen activator is determined by means of a chromogenic substrate specific for this plasminogen activator.

6. The method as claimed in claim 1, wherein the residual activity of the plasminogen activator is determined by means of plasminogen and a chromogenic plasmin substrate.

7. The method as claimed in claim 1, wherein the determination is carried out at 20-25°C.

8. An agent containing 0.1-5,000 IU/ml urokinase, 10⁻⁷-10⁻¹ g/l dextran sulfate, 5 x 10⁻⁵ to 5 mg/ml fibrinogen, 2 - 20 g/l of a degraded and chemically crosslinked gelatin, and 0.5-5 g/l of a nonionic detergent in a buffer solution of pH 6-9.

## Claims (Claims for the following Contracting State(s): ES)

1. A method for the functional determination of protein C inhibitor in a body fluid sample, which comprises prior incubation of this sample with a defined amount of a plasminogen activator, and then determination of the residual activity of the plasminogen activator, the prior incubation being carried out in the presence of dextran sulfate and fibrinogen.

2. The method as claimed in claim 1, wherein urokinase is used as plasminogen activator.

3. The method as claimed in claim 1, wherein tissue plasminogen activator is used.

4. The method as claimed in claim 1, wherein dextran sulfate is in a concentration of 10⁻⁸ to 1 mg/ml in the test batch.

5. The method as claimed in claim 1, wherein the residual activity of the plasminogen activator is determined by means of a chromogenic substrate specific for this plasminogen activator.

6. The method as claimed in claim 1, wherein the residual activity of the plasminogen activator is determined by means of plasminogen and a chromogenic plasmin substrate.

7. The method as claimed in claim 1, wherein the determination is carried out at 20-25°C.

## Revendications (Revendications pour l'(les) Etat(s) contractant(s) suivant(s): AT, BE, CH, DE, FR, GB, IT, LI, LU, NL, SE)

1. Procédé pour la détermination fonctionnelle de l'inhibiteur de protéine C dans un échantillon d'un liquide corporel, caractérisé en ce que cet échantillon est préincubé avec une quantité définie d'un activateur du plasminogène et on détermine ensuite l'activité résiduelle de l'activité du plasminogène, la préincubation étant effectuée en présence de sulfate de dextrane et de fibrinogène.

2. Procédé selon la revendication 1, caractérisé en ce que, en tant qu'activateur du plasminogène, on utilise l'urokinase.

3. Procédé selon la revendication 1, caractérisé en ce que l'on utilise l'activateur tissulaire du plasminogène.

4. Procédé selon la revendication 1, caractérisé en ce que le sulfate de dextrane est présent dans le mélange d'essai à une concentration de 10⁻⁸ à 1 mg/ml.

5. Procédé selon la revendication 1, caractérisé en ce que l'on détermine l'activité résiduelle de l'activateur du plasminogène au moyen d'un substrat chromogène spécifique de cet activateur du plasminogène.

6. Procédé selon la revendication 1, caractérisé en ce que l'on détermine l'activité résiduelle de l'activateur du plasminogène au moyen de plasminogène et d'un substrat chromogène pour la plasmine.

7. Procédé selon la revendication 1, caractérisé en ce que la détermination est effectuée à 20-25°C.

8. Agent contenant de 0,1 à 5 000 UI/ml d'urokinase, de 10⁻⁷ à 10⁻¹ g/l de sulfate de dextrane, de 5 x 10⁻⁵ à 5 mg/ml de fibrinogène, de 2 à 20 g/l d'une gélatine dégradée et réticulée chimiquement, et de 0,5 à 5 g/l d'un détergent non ionique, dans une solution tampon à pH 6-9.

## Revendications (Revendications pour l'(les) Etat(s) contractant(s) suivant(s): ES)

1. Procédé pour la détermination fonctionnelle de l'inhibiteur de protéine C dans un échantillon d'un liquide corporel, caractérisé en ce que cet échantillon est préincubé avec une quantité définie d'un activateur du plasminogène et on détermine ensuite l'activité résiduelle de l'activité du plasminogène, la préincubation étant effectuée en présence de sulfate de dextrane et de fibrinogène.

2. Procédé selon la revendication 1, caractérisé en ce que, en tant qu'activateur du plasminogène, on utilise l'urokinase.

3. Procédé selon la revendication 1, caractérisé en ce que l'on utilise l'activateur tissulaire du plasminogène.

4. Procédé selon la revendication 1, caractérisé en ce que le sulfate de dextrane est présent dans le mélange d'essai à une concentration de 10⁻⁸ à 1 mg/ml.

5. Procédé selon la revendication 1, caractérisé en ce que l'on détermine l'activité résiduelle de l'activateur du plasminogène au moyen d'un substrat chromogène spécifique de cet activateur du plasminogène.

6. Procédé selon la revendication 1, caractérisé en ce que l'on détermine l'activité résiduelle de l'activateur du plasminogène au moyen de plasminogène et d'un substrat chromogène pour la plasmine.

7. Procédé selon la revendication 1, caractérisé en ce que la détermination est effectuée à 20-25°C.
